# EUROPEAN PATENT APPLICATION

(11) **EP 4 703 724 A1**
(43) Date of publication of application: **04.03.2026**
(21) Application number: 23932293.6
(22) Date of filing: 27.04.2023
(51) Int. Cl.: G01N 33/49, G01N 21/359, A61B 5/1455, A61B 5/145, A61B 5/00

(54) **BIOMETRIC INFORMATION DETECTION DEVICE AND BIOMETRIC INFORMATION DETECTION METHOD**

(71) Applicant: Fujita Medical Instruments Co., Ltd., Tokyo 113-0033 (JP)
(72) Inventor: MAEDA Hironobu, Tokyo 1130033 (JP)
(74) Representative: Hasegawa, Kan
(86) International application number: PCT/JP2023/016697
(87) International publication number: WO 2024/224565

(57) **Abstract**

The present invention is a biological information detection device including: a first laser oscillator that oscillates a first pulsed laser beam having a suitable wavelength adapted for detection of a lactic acid or lactic acid salt; a second laser oscillator that oscillates a second pulsed laser beam having a suitable wavelength adapted for detection of a pyruvic acid; an irradiation mechanism that emits the first pulsed laser beam and the second pulsed laser beam to blood; a light receiving sensor that receives a detected laser beam output from the blood; and a control unit that controls operations of respective components. The control unit outputs oscillation instructions for oscillating the first pulsed laser beam and the second pulsed laser beam at a constant cycle, respectively, to the first laser oscillator and the second laser oscillator, cuts out detected signals provided from the light receiving sensor as time segment data for time periods corresponding to the constant cycle, respectively, and calculates respective quantities of the lactic acid or lactic acid salt and the pyruvic acid in the blood based on the time segment data.

## Description

### [Technical Field]

The present invention relates to a biological information detection device and a biological information detection method, in particular, relates to a biological information detection device and a biological information detection method for acquiring quantities of a lactic acid and a lactic acid salt present in blood.

### [Background Art]

Lactic acids contained in human blood are produced in a large amount when sugar in the blood is metabolized in an oxygen-free condition during vigorous exercise or the like, for example. The concentration of such lactic acids in blood is applied for an index of a state of shock or a circulatory failure, for example, as a value indicating the degree of blood circulation.

As an example of the above, with respect to sepsis that may damage organs of the whole body due to contamination of bacteria or the like into blood, when performing postoperative care on a patient after subjected to surgery or when transporting an emergency patient showing a symptom of shock, it is possible to determine whether or not the symptom of the patient becomes severe by monitoring the concentration of lactic acids in the blood. While measurement of such a concentration of lactic acids in blood is performed through analysis of blood drawn from the patient in general, simple and continuous measurement is difficult, and risk management against infection that may be caused by such blood drawing is essential.

As one countermeasure in this respect, for example, Patent Literature 1 discloses a measuring method including: emitting near-infrared light from a probe to a biological measuring site; receiving, at a light receiving unit, light transmitted through and scattered from a biological object; specifying a wavelength as a measuring wavelength at which light is absorbed by a lactic acid in a near-infrared region and determining the light intensity at the measuring wavelength; and performing quantitative analysis of the concentration of the lactic acid in the biological object based on the light intensity. It is disclosed that, according to this method, it is possible to noninvasively measure the lactic acid concentration and also is possible to measure a difference in lactic acid concentration depending on a measuring site without requiring any reagents.

Further, Patent Literature 2 discloses a method of monitoring a blood component level of a subject in real time, and the method includes steps of: providing a system-on-chip having a wavelength-tunable hybrid III-V/IV laser sensor; instructing the system-on-chip to monitor the blood component level of the subject by transmitting a sweep laser signal to an optical fiber interface; guiding the signal to blood of the subject at the optical fiber interface; collecting reflected signals from the blood by the optical fiber interface after the signal interacts with the blood; and guiding the reflected signals to a reflected light photodiode. In this process, the reflected signal is an optical signal; and steps of converting the reflected signal from an optical signal into an electrical signal; and converting the electrical signal into a calibrated blood component level by processing the electrical signal by a microcontroller are performed. This makes it possible to continuously collect a plurality of data points over a certain period and thereby provide important information on a history trend that may be important in evaluating effectiveness of therapy.

### [Citation List]

### [Patent Literature]

Patent Literature 1: Japanese Patent Application Laid-Open No. H9-126995
Patent Literature 2: Japanese Patent Application Laid-Open No. 2020-520768

### [Summary of Invention]

### [Technical Problem]

In the conventional arts illustrated above as an example, it may be possible to noninvasively measure the lactic acid concentration in a biological object, in particular, blood. However, the light for irradiation is emitted from a light source having a predetermined wavelength range (for example, a halogen lamp, a semiconductor laser, or the like), and thus, in particular, to selectively and accurately detect a lactic acid, it is required to perform calibration for selecting a wavelength or a wavelength band suitable for measurement of the lactic acid out of a light signal received at the light receiving unit. If such calibration is not properly performed, the accuracy of a measurement result will be significantly reduced.

Further, in addition to lactic acids, pyruvic acids whose chemical structure and absorbance characteristics are similar to the lactic acids are also present in blood. As mentioned previously, since lactic acids and pyruvic acids have similar chemical structure and similar absorbance characteristics, there is a high likelihood of inclusion of a pyruvic acid together with a lactic acid in a light signal detected at a wavelength range used for measuring the lactic acid as with the conventional art.

In view of such circumstances, the present application intends to provide a biological information detection device and a biological information detection method that can acquire concentrations of a lactic acid and a pyruvic acid contained in blood independently of each other.

### [Solution to Problem]

A biological information detection device according to one aspect of the present invention includes: a first laser oscillator that oscillates a first pulsed laser beam having a suitable wavelength adapted for detection of a lactic acid or lactic acid salt; a second laser oscillator that oscillates a second pulsed laser beam having a suitable wavelength adapted for detection of a pyruvic acid; an irradiation mechanism that emits the first pulsed laser beam and the second pulsed laser beam to blood inside which the lactic acid or lactic acid salt and the pyruvic acid are present; a light receiving sensor that receives a detected laser beam output from the blood; and a control unit that controls operations of respective components. The control unit outputs oscillation instructions for oscillating the first pulsed laser beam and the second pulsed laser beam at a constant cycle, respectively, to the first laser oscillator and the second laser oscillator, cuts out detected signals provided from the light receiving sensor as time segment data for time periods corresponding to the constant cycle, and calculates respective quantities of the lactic acid or lactic acid salt and the pyruvic acid in the blood based on the time segment data.

Further, a biological information detection method according to another aspect of the present invention is a biological information detection method including: emitting a first pulsed laser beam, which has a suitable wavelength adapted for detection of a lactic acid or lactic acid salt, and a second pulsed laser beam, which has a suitable wavelength adapted for detection of a pyruvic acid, to blood inside which the lactic acid or lactic acid salt and the pyruvic acid are present; receiving detected laser beams output from the blood at a light receiving sensor; and acquiring quantities of the lactic acid or lactic acid salt and the pyruvic acid in the blood, respectively. The first pulsed laser beam and the second pulsed laser beam are oscillated at a constant cycle, respectively, and the biological information detection method includes: cutting out, for each of the suitable wavelength of the first pulsed laser beam and the suitable wavelength of the second pulsed laser beam, detected signals provided from the light receiving sensor as time segment data for time periods corresponding to the constant cycle; and calculating respective quantities of the lactic acid or lactic acid salt and the pyruvic acid in the blood based on the time segment data.

### [Advantageous Effects of Invention]

According to one aspect of the present invention, concentrations of a lactic acid and a pyruvic acid contained in blood can be acquired independently of each other. This makes it possible to more accurately know quantities of the lactic acid and lactic acid salt contained in blood.

### [Brief Description of Drawings]

[Fig. 1] Fig. 1 is a schematic diagram illustrating a configuration of a biological information detection device according to a first embodiment that is a representative example of the present invention.
[Fig. 2] Fig. 2 is a block diagram illustrating an example of a configuration of a laser oscillator included in the biological information detection device illustrated in Fig. 1.
[Fig. 3] Fig. 3 is a partial sectional view illustrating an example of a configuration of an irradiation mechanism included in the biological information detection device illustrated in Fig. 1.
[Fig. 4] Fig. 4 is a block diagram illustrating an example a configuration of a control unit included in the biological information detection device illustrated in Fig. 1.
[Fig. 5] Fig. 5 is a flowchart illustrating an overview of a biological information detection method according to the first embodiment.
[Fig. 6A] Fig. 6A is a plan view illustrating an overview of an operation procedure in which blood is irradiated with a pulsed laser beam in the biological information detection device according to the first embodiment.
[Fig. 6B] Fig. 6B is a partial front view illustrating an overview of an operation procedure in which blood is irradiated with a pulsed laser beam in the biological information detection device according to the first embodiment.
[Fig. 6C] Fig. 6C is a partial front view illustrating an overview of an operation procedure in which blood is irradiated with a pulsed laser beam in the biological information detection device according to the first embodiment.
[Fig. 7] Fig. 7 is a time-series graph illustrating a relationship between various instruction signals and measurement data.
[Fig. 8A] Fig. 8A is a plan view illustrating an overview of an operation procedure in which blood is irradiated with a pulsed laser beam in a biological information detection device according to a second embodiment.
[Fig. 8B] Fig. 8B is a partial front view illustrating an overview of an operation procedure in which blood is irradiated with a pulsed laser beam in the biological information detection device according to the second embodiment.
[Fig. 8C] Fig. 8C is a partial front view illustrating an overview of an operation procedure in which blood is irradiated with a pulsed laser beam in the biological information detection device according to the second embodiment.
[Fig. 9] Fig. 9 is a flowchart illustrating an overview of a biological information detection method according to the second embodiment.
[Fig. 10A] Fig. 10A is a plan view illustrating an overview of an operation procedure in which blood is irradiated with a pulsed laser beam in a biological information detection device according to a third embodiment.
[Fig. 10B] Fig. 10B is a partial front view illustrating an overview of an operation procedure in which blood is irradiated with a pulsed laser beam in the biological information detection device according to the third embodiment.
[Fig. 10C] Fig. 10C is a partial front view illustrating an overview of an operation procedure in which blood is irradiated with a pulsed laser beam in the biological information detection device according to the third embodiment.
[Fig. 11] Fig. 11 is a time-series graph illustrating a relationship between various instruction signals and measurement data.
[Fig. 12] Fig. 12 is a partial sectional view illustrating an overview of a measuring unit according to a modified example for the third embodiment.

### [Description of Embodiments]

Embodiments of the biological information detection device and the biological information detection method according to representative examples of the present invention will be described below with reference to the drawings.

### <First Embodiment>

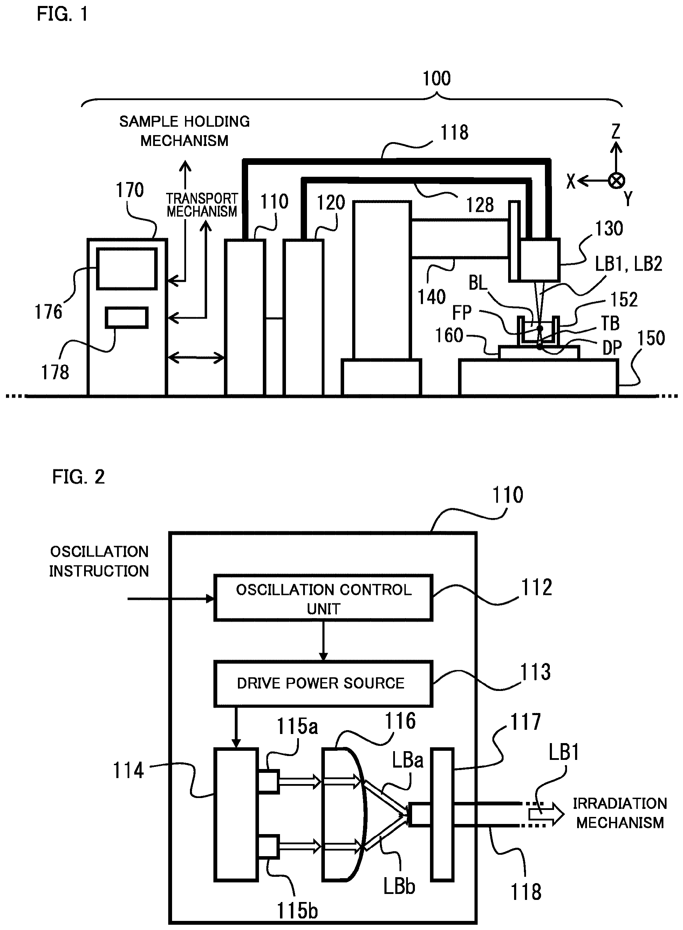

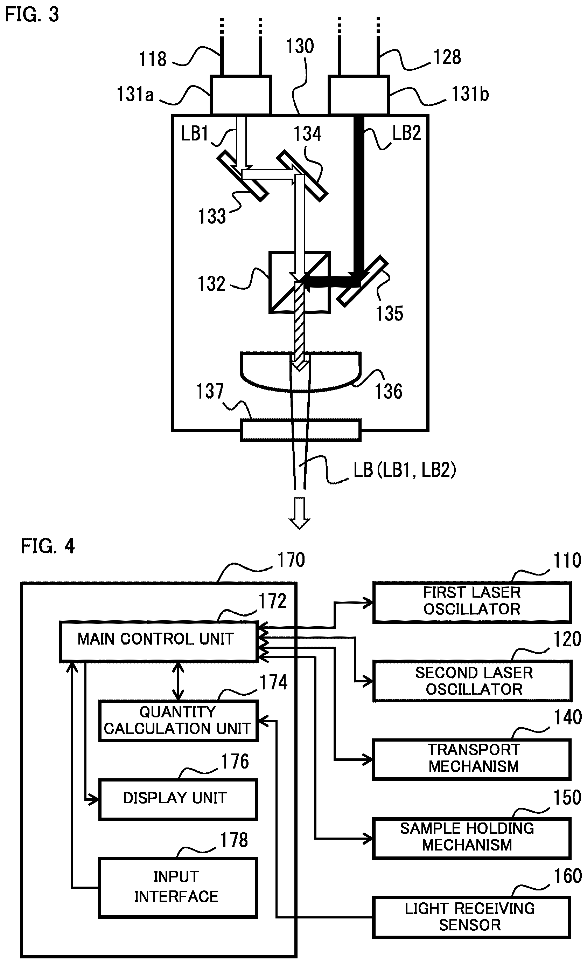

Fig. 1 is a schematic diagram illustrating a configuration of a biological information detection device according to a first embodiment that is a representative example of the present invention. Further, Fig. 2 is a block diagram illustrating an example of a configuration of a laser oscillator included in the biological information detection device illustrated in Fig. 1. Further, Fig. 3 is a partial sectional view illustrating an example of a configuration of an irradiation mechanism included in the biological information detection device illustrated in Fig. 1. Furthermore, Fig. 4 is a block diagram illustrating an example a configuration of a control unit included in the biological information detection device illustrated in Fig. 1.

As illustrated in Fig. 1, a biological information detection device 100 according to the first embodiment includes, as an example thereof, a first laser oscillator 110 that oscillates a first pulsed laser beam LB1 having a suitable wavelength adapted for detection of a lactic acid or lactic acid salt, a second laser oscillator 120 that oscillates a second pulsed laser beam LB2 having a suitable wavelength adapted for detection of a pyruvic acid, an irradiation mechanism 130 that emits the first pulsed laser beam LB1 and the second pulsed laser beam LB2 to blood BL inside which a lactic acid or lactic acid salt and a pyruvic acid are present, a transport mechanism 140 that moves the irradiation mechanism 130 to any positions in X, Y, and Z directions, a sample holding mechanism 150 that moves positions in the X, Y, and Z directions of a container 152 containing the blood BL, a light receiving sensor 160 that receives a detected laser beam output from the blood BL, and a control unit 170 that controls operations of respective components.

Note that, in the following description, the lactic acid or lactic acid salt and the pyruvic acid contained in the blood BL are labeled with a reference "MM" collectively as "measuring target substance" (see a reference MM in Fig. 4 and the like described later).

A light source that outputs a wavelength suitable for detecting a lactic acid or lactic acid salt out of the measuring target substance MM contained in the blood BL (for example, a wavelength having high absorbance efficiency or the like) is applied to the first laser oscillator 110. Herein, as the suitable wavelength of the pulsed laser beam LB when detecting a lactic acid or lactic acid salt, 1480 nm is employed as an example thereof.

A light source that outputs a wavelength suitable for detecting a pyruvic acid out of the measuring target substance MM contained in the blood BL (for example, a wavelength having high absorbance efficiency or the like) is applied to the second laser oscillator 120. Herein, as the suitable wavelength of the pulsed laser beam LB when detecting a pyruvic acid, 1462 nm is employed as an example thereof.

The first laser oscillator 110 includes, as illustrated in Fig. 2 as an example thereof, an oscillation control unit 112 that performs control to oscillate the first pulsed laser beam LB1 at a constant cycle T based on an oscillation instruction signal from the control unit 170, a drive power source 113 that supplies drive power to a plurality of laser sources 115a, 115b in response to an ON/OFF signal from the oscillation control unit 112, a support part 114 to which the laser sources 115a, 115b are attached, a condenser lens 116 that focuses the pulsed laser beams LBa, LBb emitted from the plurality of laser sources 115a, 115b, a wavelength adjustment unit 117 that adjusts the wavelength of the focused first pulsed laser beam LB1, and a transmission path 118 (for example, an optical fiber) that transmits the focused first pulsed laser beam LB1 to the irradiation mechanism 130.

Herein, although Fig. 2 illustrates the case as an example where two laser sources LBa, LBb are used as the light source of the first pulsed laser beam LB1, it is also possible to provide three or more laser sources in an arrayed manner or on a predetermined circumference. Further, light emitting diodes (LED), semiconductor lasers (LD), or the like can be employed as the plurality of laser sources 115a, 115b that selectively emit suitable wavelengths in order to detect a lactic acid or lactic acid salt and a pyruvic acid described above.

Further, the pulsed laser beams LBa, LBb emitted from the plurality of laser sources 115a, 115b are focused coaxially to increase power, the coaxially focused pulsed laser beams LBa, LBb are transmitted through the wavelength adjustment unit 117, and thereby the first pulsed laser beam LB1 whose wavelength has been selected in a predetermined suitable wavelength range is emitted into the transmission path 118. Herein, as the wavelength adjustment unit 117, a band-pass filter that selectively removes light whose wavelength is outside a predetermined range between the upper limit and the lower limit, or the like may be illustrated as an example.

Note that, although the case where drive power is directly supplied to the plurality of laser sources 115a, 115b from the drive power source 113 has been illustrated, an amplifier circuit (not illustrated) may be provided to the support part 114, for example, and drive power from the drive power source 113 may be amplified and then supplied to the laser sources 115a, 115b. Further, since the configuration of the second laser oscillator 120 may employ the same configuration as that of the first laser oscillator 110 described above, the description thereof will be omitted.

The irradiation mechanism 130 includes, as illustrated in Fig. 3 as an example thereof, connectors 131a, 131b connected to the transmission paths 118, 128 that transmit the first pulsed laser beam LB1 and the second pulsed laser beam LB2, respectively, a beam splitter 132 that guides the transmitted first pulsed laser beam LB1 and second pulsed laser beam LB2 to a coaxial light path, mirrors 133, 134 that reflect and guide the first pulsed laser beam LB1 to the beam splitter 132, a mirror 135 that reflects and guides the second pulsed laser beam LB2 to the beam splitter 132, a condenser lens 136 that focuses the coaxially guided pulsed laser beam LB (that is, a laser beam composed of the superimposed first pulsed laser beam LB1 and second pulsed laser beam LB2) at a predetermined focused position, and a window member 137 through which the pulsed laser beam LB is transmitted.

The first pulsed laser beam LB1 and the second pulsed laser beam LB2 introduced to the irradiation mechanism 130 via the transmission paths 118, 128 are reshaped by the condenser lens 136 into a beam profile of a predetermined beam diameter, a predetermined beam sectional shape, and the like and then emitted from the window member 137 to the blood BL contained in the container 152. Herein, although the case where the pulsed laser beam LB is reshaped to have a beam spot of a circular cross section has been illustrated as an example in the first embodiment, the beam profile can also be reshaped into any shape such as a polygonal or linear shape with suitable selection of the condenser lens.

Note that, although the beam splitter 132 has been illustrated as an example of an optical element that guides the first pulsed laser beam LB1 and the second pulsed laser beam LB2 into a coaxial optical path in the configuration illustrated in Fig. 3, a different optical element may be applied as long as it guides (emits) two laser beams coaxially. Further, although the window member 137 is used as a member for holding airtightness inside the irradiation mechanism 130, a so-called "telecentric fθ lens" may be added to the window member 137.

The transport mechanism 140 is configured as a linear driver as an example thereof that moves relatively in three axis directions of X, Y, and Z orthogonal to each other, and the irradiation mechanism 130 is attached to one end of the transport mechanism 140. Note that the transport mechanism 140 may be configured as a six-axis or seven-axis type industrial robot having a robot arm whose one end has the irradiation mechanism 130 attached thereto.

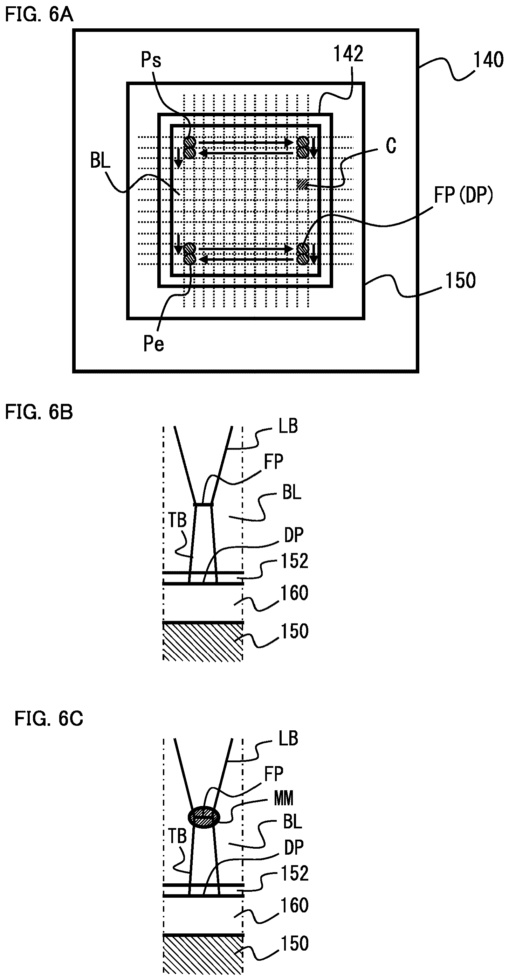

The sample holding mechanism 150 is configured as a table as an example thereof that is movable in the three axis directions of X, Y, and Z in the depiction while the container 152, which contains the blood BL containing the measuring target substance MM, is placed on the top face. Further, the light receiving sensor 160 to detect a transmitted beam TB (see Fig. 6B, Fig. 6C, and the like) from the blood BL is arranged between the top face of the sample holding mechanism 150 and the bottom face of the container 152.

Note that the container 152 containing the blood BL is made of a material that is transparent to the wavelength of the pulsed laser beam LB described above (that is, through which the pulsed laser beam LB on irradiation transmits). Accordingly, the pulsed laser beam LB with which the blood BL contained in the container 152 is irradiated transmits directly through the bottom surface of the container 152 in a region where no measuring target substance MM is present and then reaches the light receiving sensor 160, and the irradiation of the pulsed laser beam LB is thus detected by the light receiving sensor 160.

The control unit 170 includes, as illustrated in Fig. 4 as an example thereof, a main control unit 172 that outputs operation instructions to respective components of the biological information detection device 100, a quantity calculation unit 174 that calculates respective quantities of a lactic acid or lactic acid salt and a pyruvic acid in the measuring target substance MM contained in the blood BL by using detected values from the light receiving sensor 160, a display unit 176 that displays the calculated quantities of the lactic acid or lactic acid salt and the pyruvic acid, other various parameters, or the like, and an input interface 178 that enables manual input of information for correcting various parameters such as measurement conditions. Further, in the control unit 170, the main control unit 172 is connected to the first laser oscillator 110, the second laser oscillator 120, the transport mechanism 140, and the sample holding mechanism 150 via a wired or wireless connection and transfers signals to and from these peripheral equipments to control the operation of the entire biological information detection device 100.

The main control unit 172 has, as an example thereof, a function of, in response to a start signal Ss corresponding to start of measurement being input via the input interface 178 from the user, extracting operation information on oscillation for the first laser oscillator 110 and the second laser oscillator 120, operation information on relative motion for the transport mechanism 140 and the sample holding mechanism 150, or the like provided from a predetermined measurement program and then generating and outputting oscillation signals S1o, S2o and a relative motion signal Sm used for implementing the above operations to respective components. Further, the main control unit 172 also has a function of outputting the first oscillation signal S1o and the second oscillation signal S2o to the quantity calculation unit 174 described later in synchronization with the output to the first laser oscillator 110 and the second laser oscillator 120 and, in response to a calculation result from the quantity calculation unit 174, transmitting the calculation result of the quantities or the current various parameters of the biological information detection device 100 to the display unit 176 to cause them to be displayed.

As described above, the quantity calculation unit 174 has a function of, in response to receiving a calculation signal Se corresponding to start of calculation from the main control unit 172 that has received the start signal SS described above, continuously receiving and accumulating a detected signal Sd corresponding to a detected value at time t from the light receiving sensor 160. Further, the quantity calculation unit 174 also has a function of receiving the first oscillation signal S1o and the second oscillation signal S2o from the main control unit 172, cutting time segment data D associated with respective oscillation timings of the first laser oscillator 110 and the second laser oscillator 120 out from time-series data of the detected signal Sd detected by the light receiving sensor 160, and calculating the quantities of a lactic acid or lactic acid salt and a pyruvic acid in the measuring target substance MM contained in the blood BL based on the time segment data D. The results of quantities of the lactic acid or lactic acid salt and the pyruvic acid are then transmitted to the main control unit 172.

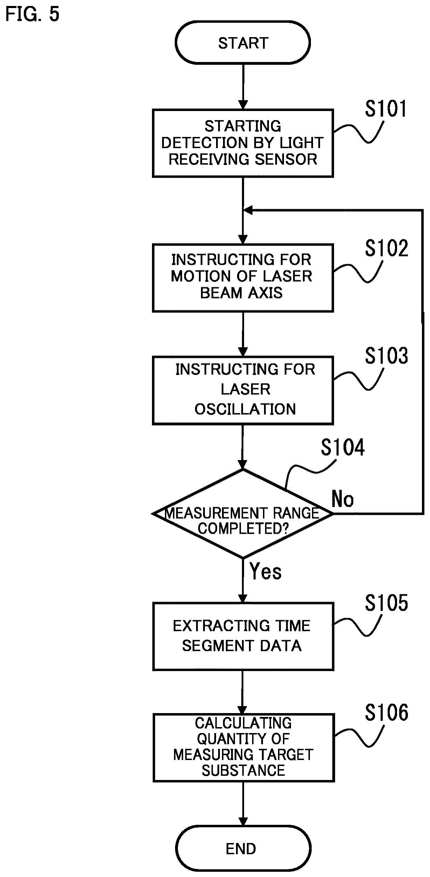

Next, a specific operation form of the biological information detection method performed by the biological information detection device according to the first embodiment will be described with reference to Fig. 5 to Fig. 7.

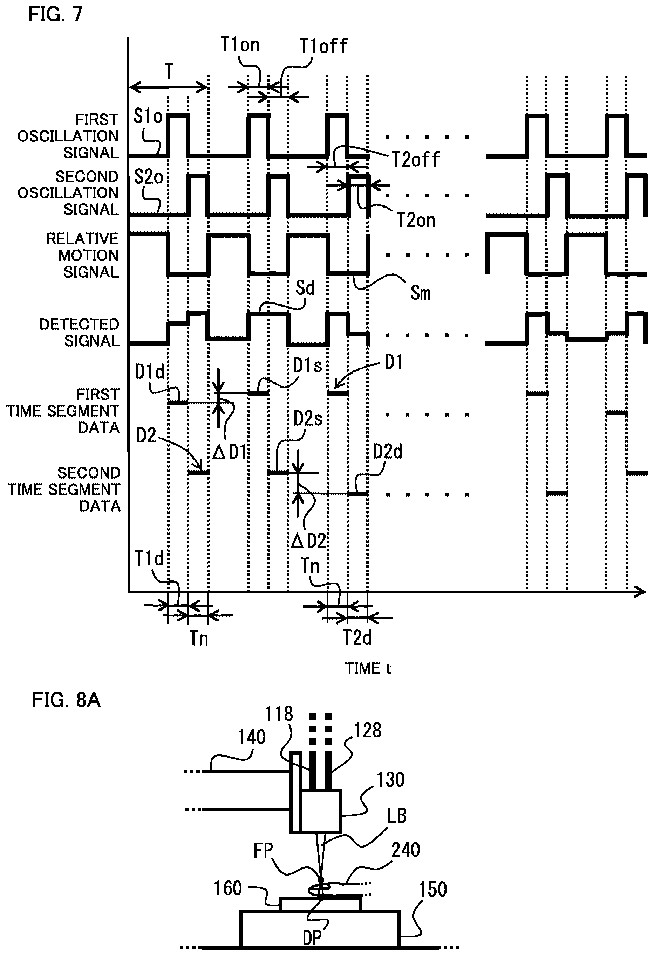

Fig. 5 is a flowchart illustrating the overview of the biological information detection method according to the first embodiment. Further, Fig. 6A to Fig. 6C are a plan view and partial front views illustrating the overview of the operation procedure in which blood is irradiated with a pulsed laser beam in the biological information detection device according to the first embodiment. Furthermore, Fig. 7 is a time-series graph illustrating the relationship between various instruction signals and measurement data.

In the biological information detection method performed by the biological information detection device 100 according to the first embodiment, as illustrated in Fig. 5, in response to the start signal Ss corresponding to start of measurement being input via the input interface 178 from the user, the main control unit 172 of the control unit 170 first instructs the quantity calculation unit 174 to start receiving the detected signal Sd from the light receiving sensor 160 (step S101). Accordingly, in the quantity calculation unit 174, the detected signal Sd from the light receiving sensor 160 is continuously received and temporarily stored as time-series data until the end of the operation indicated by the flowchart.

Subsequently, the main control unit 172 outputs the relative motion signal Sm to the transport mechanism 140 and the sample holding mechanism 150 based on a predetermined measurement program (step S102). Accordingly, the position and the focus distance applied when the blood BL is irradiated with the pulsed laser beam LB are positioned.

Next, the main control unit 172 outputs, to the first laser oscillator 110 and the second laser oscillator 120, the first oscillation signal S1o and the second oscillation signal S2o to emit the first pulsed laser beam LB1 or the second pulsed laser beam LB2 for a defined irradiation time period Ton (step S103). In the first laser oscillator 110 that has received the first oscillation signal S1o, the oscillation control unit 112 outputs an ON-instruction signal Son during the irradiation time period Ton described above to the drive power source 113, and the first pulsed laser beam LB1 adjusted at a predetermined wavelength is emitted. Note that this operation is also performed similarly in parallel in the second laser oscillator 120.

Subsequently, the main control unit 172 determines based on the above measurement program whether or not emission of the pulsed laser beam LB for all the measurement ranges defined for the blood BL in the container 152 is completed (step S104) . That is, if it is determined in step S104 that the irradiation for all the measurement ranges is completed, the main control unit 172 outputs the calculation signal Se to the quantity calculation unit 174 indicating the completion of measurement on the blood BL and proceeds to the subsequent step S105.

In contrast, if it is determined in step S104 that the irradiation in all the measurement ranges is not completed, the process returns to step S102, and positioning and emission of the pulsed laser beam LB for uncompleted measurement range are repeatedly performed in accordance with the measurement program. Accordingly, detection of the measuring target substance MM is performed for all the ranges (regions) where the blood BL contained in the container 152 is to be measured.

Fig. 6A to Fig. 6C illustrate a specific example for the operation procedure from step S102 to step S104 described above. That is, as illustrated in Fig. 6A, the blood BL is contained in the container 152 placed on the light receiving sensor 160 having a sensing surface on the top side, and a plurality of rectangular regions C having vertical and horizontal lengths corresponding to the focus diameter (spot diameter) of a focus point FP of the pulsed laser beam LB are defined in the blood BL.

Further, for the plurality of rectangular regions C defined as described above, an irradiation start position Ps and an irradiation end position Pe of the pulsed laser beam LB are further defined by the measurement program, and a scan path for scanning a part between the irradiation start position Ps and the irradiation end position Pe in the XY direction is defined. Note that, as an example, in the determination in step S104 of the flowchart illustrated in Fig. 5, the determination is made in accordance with whether or not the current irradiation position (focus point FP) matches the irradiation end position Pe on the scan path.

Next, the relationship between the presence or absence of the measuring target substance MM and a detected signal from the light receiving sensor 160 at an irradiation position (focus point FP) of the pulsed laser beam LB will be described below. For example, as illustrated in Fig. 6B, when the measuring target substance MM is not present at the focus point FP of the pulsed laser beam LB on irradiation or on the extension line therefrom, since the pulsed laser beam LB transmits through the blood BL and the container 152, a transmitted beam TB corresponding to the power of the emitted pulsed laser beam LB is detected at a light receiving point DP of the light receiving sensor 160.

In contrast, as illustrated in Fig. 6C, when the measuring target substance MM is present at the focus point FP of the pulsed laser beam LB on irradiation or on the extension line therefrom, since the first pulsed laser beam LB1 or the second pulsed laser beam LB2 emitted as the pulsed laser beam LB is absorbed or reflected by the lactic acid or lactic acid salt and the pyruvic acid in the measuring target substance MM, a transmitted beam TB having power lower than the power of the emitted pulsed laser beam LB is detected at the light receiving point DP of the light receiving sensor 160. Note that, although the case where the measuring target substance MM is larger than the focus diameter at the focus point FP of the pulsed laser beam LB is illustrated as an example in Fig. 6C, the same tendency is exhibited even when the measuring target substance MM is smaller than the focus diameter.

Subsequently, as illustrated in Fig. 7, the quantity calculation unit 174 that has received the calculation signal Se from the main control unit 172 cuts out time segments of the detected signal Sd provided from the light receiving sensor 160 corresponding to respective time segments in which the first oscillation signal S1o and the second oscillation signal S2o have been received from the main control unit 172 and thereby extracts the first time segment data D1 corresponding to the first pulsed laser beam LB1 and the second time segment data D2 corresponding to the second pulsed laser beam LB2 (step S105). Accordingly, for the detected signal Sd, it is possible to refine data to only the data on the time segment corresponding to an irradiation time period T1on during which the first pulsed laser beam LB1 has been emitted or the time segment corresponding to an irradiation time period T2on during which the second pulsed laser beam LB2 has been emitted (that is, it is possible to reduce noise during detection). Herein, a sum of an irradiation time period T1on and a non-irradiation time period T1off of the first oscillation signal S1o is defined as one cycle T (the same applies to the relationship between the second oscillation signal S2o and one cycle T).

Next, the quantity calculation unit 174 calculates the quantities of the lactic acid or lactic acid salt and the pyruvic acid in the measuring target substance MM to the blood BL based on the extracted first time segment data D1 and second time segment data D2. Specifically, the first time segment data D1 extracted in step S105 includes, as an example thereof, two levels of output values, namely, reference data D1s (the state illustrated in Fig. 6B) when the measuring target substance MM is not detected (a non-detection time segment Tn)and detection data D1d (the state illustrated in Fig. 6C) when the measuring target substance MM is detected (a detection time segment T1d).

Herein, it can be determined that the larger the absolute value of a difference ΔD1 between the reference data D1s and the detection data D1d is, the greater the detected amount of the measuring target substance MM is. Accordingly, after the measurement for all the measurement ranges of interest, the quantity calculation unit 174 accumulates the number of detection data D1d in the entire time segment data D1, outputs the accumulated number to the main control unit 172 as the "quantity" of the measuring target substance MM, and ends the operation (step S106).

On the other hand, the extracted second time segment data D2 includes, as an example thereof, two levels of output values, namely, reference data D2s when the measuring target substance MM is not detected (a non-detection time segment Tn) and detection data D2d when the measuring target substance MM is detected (a detection time segment T2d). Therefore, in the same manner as in the case of the first time segment data D1, it can be determined that the larger the absolute value of a difference ΔD2 between the reference data D2s and the detection data D2d is, the greater the detected amount of the measuring target substance MM is.

Note that the quantity of the measuring target substance MM may be calculated as a ratio relative to the total, instead of the accumulated number. Further, a predetermined threshold may be provided for the difference ΔD1 or ΔD2 between the reference data and the detection data, and an instance that exceeds the predetermined threshold may be determined as "detected".

With the configuration as described above, the biological information detection device and the biological information detection method according to the first embodiment are configured to output oscillation instructions to two laser oscillators having wavelengths suitable for detecting a lactic acid or lactic acid salt and a pyruvic acid so as to oscillate pulsed laser beams at a constant cycle, cut out a detected signal provided from a light receiving sensor as time segment data corresponding to the laser beam at each wavelength in the time period corresponding to that constant cycle, and calculate the quantities of the lactic acid or lactic acid salt and the pyruvic acid contained in blood based on the time segment data. Thus, the concentrations of the lactic acid and the pyruvic acid contained in blood can be acquired independently of each other.

### <Second Embodiment>

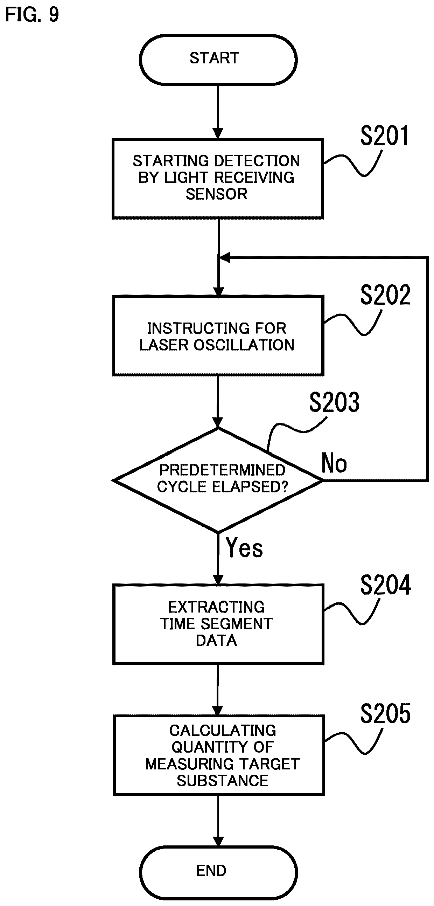

Next, an embodiment of a biological information detection device and a biological information detection method according to a second embodiment that is another example of the present invention will be described with reference to Fig. 8A to Fig. 8C and Fig. 9. Note that, in the second embodiment, components that may employ features identical or common to those of the first embodiment in the schematic diagrams or the like illustrated in Fig. 1 to Fig. 7 are labeled with the same references, and the repeated description thereof will be omitted.

Fig. 8A to Fig. 8C are a plan view and partial front views illustrating the overview of the operation procedure in which blood is irradiated with a pulsed laser beam in the biological information detection device according to the second embodiment. Furthermore, Fig. 9 is a flowchart illustrating the overview of the biological information detection method according to the second embodiment.

The biological information detection device 100 according to the second embodiment uses the scheme to irradiate a blood vessel or the like with the pulsed laser beam LB for direct measurement and perform measurement while blood BL flows inside an organism such as a human, for example, in contrast to the measuring scheme using the container 152 containing the blood BL in the first embodiment. That is, as illustrated in Fig. 8A, a terminal part of a human body (a finger 240 or the like), for example, through which the irradiated pulsed laser beam LB is relatively likely to transmit is placed on the light receiving sensor 160 having a sensing surface on the top side, and in this state, the pulsed laser beam LB is emitted toward the finger 240.

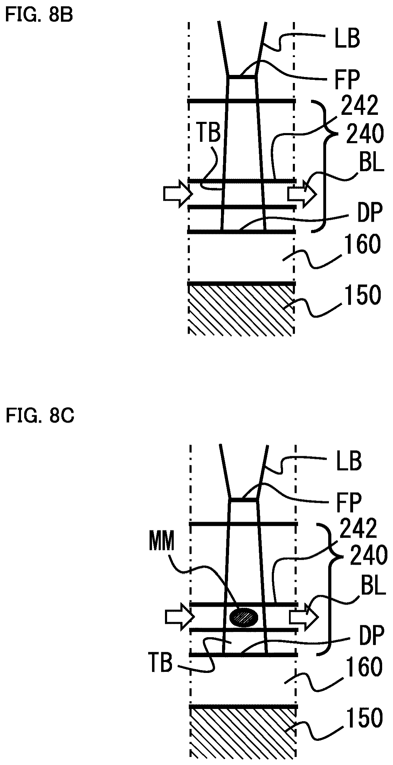

At this time, at the irradiation position (focus point FP) of the pulsed laser beam LB, as illustrated in Fig. 8B, for example, when the measuring target substance MM is not present at the focus point FP of the pulsed laser beam LB on irradiation or on the extension line therefrom, since the pulsed laser beam LB transmits through the finger 240 including the blood vessel 242, the transmitted beam TB corresponding to the power of the emitted pulsed laser beam LB is detected at a light receiving point DP of the light receiving sensor 160.

In contrast, as illustrated in Fig. 8C, when the measuring target substance MM is present at the focus point FP of the pulsed laser beam LB irradiated or on the extension line therefrom, since the pulsed laser beam LB is absorbed or reflected by a lactic acid or lactic acid salt and a pyruvic acid in the measuring target substance MM, a transmitted beam TB having power lower than the power of the emitted pulsed laser beam LB is detected at the light receiving point DP of the light receiving sensor 160 in the same manner as in the first embodiment. Note that, also in Fig. 8C, the same tendency is exhibited even when the measuring target substance MM is smaller than the focus diameter of the focus point FP of the pulsed laser beam LB in the same manner as in the case of the first embodiment.

In the state of such arrangement, while the pulsed laser beam LB in accordance with ON/OFF control at the constant cycle T is being emitted for a predetermined time period, the detected signal Sd from the light receiving sensor 160 is received. Accordingly, instead of scanning and measuring a measurement region of the blood BL in the container 152 as in the first embodiment, it is possible to measure time-series data on the blood BL as blood that is continuously flowing without moving the optical axis of the pulsed laser beam LB (that is, the irradiation mechanism 130).

In the biological information detection method according to the second embodiment, as illustrated in Fig. 9, in response to the start signal Ss corresponding to start of measurement being input via the input interface 178 from the user, the main control unit 172 of the control unit 170 first instructs the quantity calculation unit 174 to start receiving the detected signal Sd from the light receiving sensor 160 (step S201). Accordingly, in the same manner as in the case of the first embodiment, in the quantity calculation unit 174, the detected signal Sd from the light receiving sensor 160 is continuously received and temporarily stored as time-series data until the end of the operation indicated by the flowchart.

Subsequently, the main control unit 172 outputs, to the first laser oscillator 110 and the second laser oscillator 120, the first oscillation signal S1o and the second oscillation signal S2o to emit the pulsed laser beam LB for a defined irradiation time period Ton based on a predetermined measurement program (step S202). In the first laser oscillator 110 that has received the first oscillation signal S1o, in the same manner as in the first embodiment, the oscillation control unit 112 outputs an ON-instruction signal Son to the drive power source 113 for the irradiation time period Ton described above, and the pulsed laser beam LB adjusted at a predetermined wavelength is emitted. Note that this operation is also performed similarly in parallel in the second laser oscillator 120.

Subsequently, the main control unit 172 determines based on the above measurement program whether or not the emission of the pulsed laser beam LB at the constant cycle T is completed for predetermined cycles (step S203). That is, if it is determined in step S203 that the emission of a predetermined number of cycles is completed, the main control unit 172 outputs the calculation signal Se to the quantity calculation unit 174 indicating the completion of measurement on the blood BL and proceeds to the subsequent step S204.

In contrast, if it is determined in step S203 that the emission of a predetermined number of cycles is not completed, the process returns to step S202, and emission of the pulsed laser beam LB for one cycle is repeated. Accordingly, the detection operation on the measuring target substance is performed over a predetermined time period for the blood BL flowing continuously in the blood vessel 242 of the finger 240.

Subsequently, in the same manner as in the first embodiment, the quantity calculation unit 174 that has received the calculation signal Se from the main control unit 172 cuts out respective time segments of the detected signal Sd provided from the light receiving sensor 160 corresponding to time segments in which the first oscillation signal S1o and the second oscillation signal S2o have been received from the main control unit 172, respectively, and thereby extracts the first time segment data D1 and the second time segment data D2 (step S204). Accordingly, it is possible to refine data to only the data on the time segments during which the first pulsed laser beam LB1 and the second pulsed laser beam LB2 have been emitted for the detected signal Sd, respectively.

Next, the quantity calculation unit 174 calculates the quantities of the lactic acid or lactic acid salt and the pyruvic acid in the measuring target substance MM to the blood BL based on the extracted first time segment data D1 and second time segment data D2 in the same manner as in the case of the first embodiment. The quantity calculation unit 174 then outputs the calculated quantity of the lactic acid or lactic acid salt and the calculated quantity of the pyruvic acid to the main control unit 172 and ends the operation (step S205).

With the configuration as described above, the biological information detection device and the biological information detection method according to the second embodiment use the scheme to irradiate a blood vessel of a finger or the like with a pulsed laser beam for direct measurement and perform measurement while blood flows inside an organism such as a human, for example. Thus, in addition that the advantageous effects described in the first embodiment can be obtained, it is not required to acquire blood in advance from a human body or the like, and this can reduce the burden during measurement. Further, since the step of relatively moving the pulsed laser beam with respect to the container containing the blood to be measured is not required, the overall measuring time can also be shortened.

### <Third Embodiment>

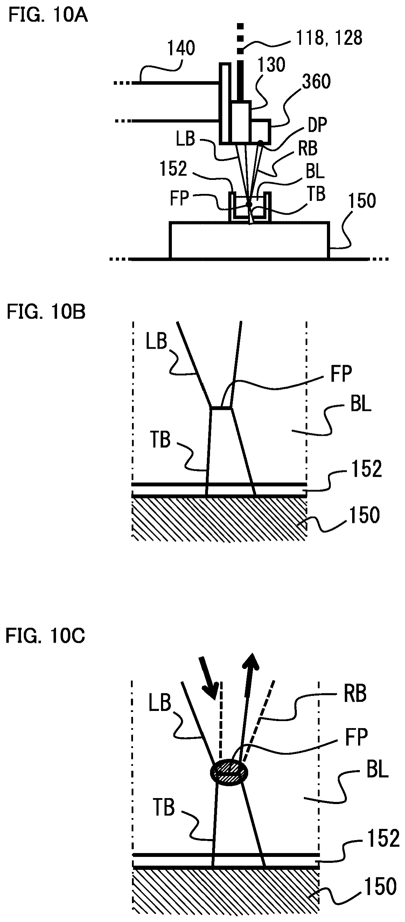

Next, an embodiment of a biological information detection device and a biological information detection method according to a third embodiment that is yet another example of the present invention will be described with reference to Fig. 10A to Fig. 10C, Fig. 11, and Fig. 12. Note that, in the third embodiment, components that may employ features identical or common to those of the first embodiment and the second embodiment in the schematic diagrams or the like illustrated in Fig. 1 to Fig. 9 are labeled with the same references, and the repeated description thereof will be omitted.

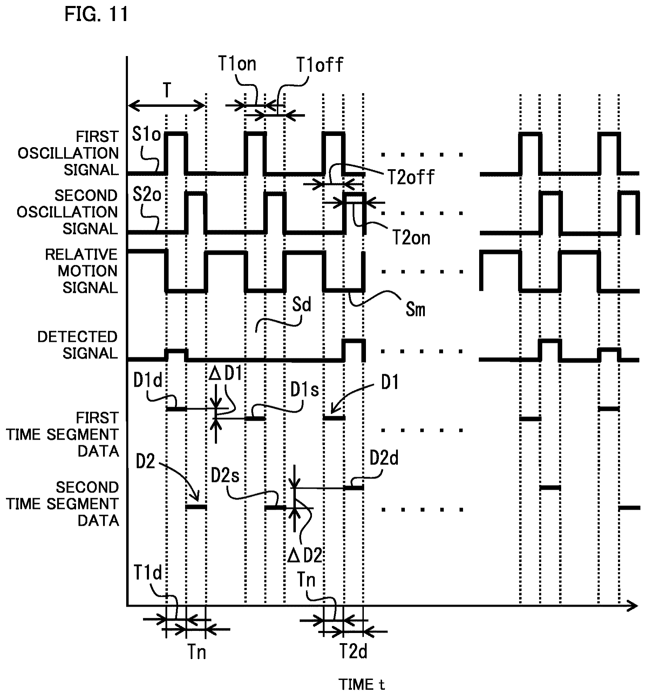

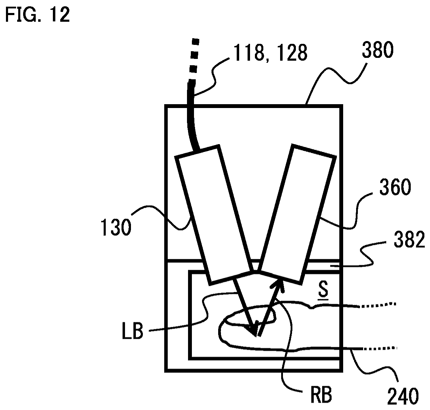

Fig. 10A to Fig. 10C are a plan view and partial front views illustrating the overview of the operation procedure in which blood is irradiated with a pulsed laser beam in the biological information detection device according to the third embodiment. Further, Fig. 11 is a time-series graph illustrating a relationship between various instruction signals and measurement data. Furthermore, Fig. 12 is a partial sectional view illustrating the overview of a measuring unit according to a modified example for the biological information detection device according to the third embodiment.

The biological information detection device 100 according to the third embodiment uses a scheme to measure a reflected beam RB from the measuring target substance MM contained in the blood BL in contrast to the measuring scheme to detect a transmitted beam TB from the blood BL by using the light receiving sensor 160 as in the first embodiment. That is, as illustrated in Fig. 10A, the blood BL is contained in the container 152 placed on the sample holding mechanism 150, and the pulsed laser beam LB is emitted to a predetermined measuring range of the blood BL in the same manner as in the case of the first embodiment.

At this time, in the biological information detection device 100 according to the third embodiment, the reflected beam RB reflected by the measuring target substance MM contained in the blood BL is detected by the light receiving sensor 360 attached to the irradiation mechanism 130. That is, as illustrated in Fig. 10B, for example, when the measuring target substance MM is not present at the focus point FP of the pulsed laser beam LB on irradiation or on the extension line therefrom, since the pulsed laser beam LB transmits through the blood BL and the container 152, only the detected value based on the amount of light around the device is detected by the light receiving sensor 360.

In contrast, as illustrated in Fig. 10C, when the measuring target substance MM is present at the focus point FP of the pulsed laser beam LB irradiated or on the extension line therefrom, since the pulsed laser beam LB is absorbed or reflected by the measuring target substance MM, the reflected beam RB that is a part of the output of the pulsed laser beam LB irradiated is detected at the light receiving point DP of the light receiving sensor 360. Note that, in Fig. 10C, the same tendency is exhibited even when the measuring target substance MM is smaller than the focus diameter of the focus point FP of the pulsed laser beam LB in the same manner as in the case of the first embodiment.

In the biological information detection device 100 according to the third embodiment, as illustrated in Fig. 11, the detected signal Sd that gives a detected value of a predetermined amount of light is received in a time segment in which the light receiving sensor 360 has detected the reflected beam RB. Then, in the same manner as in the first embodiment, the quantity calculation unit 174 cuts out the time segment of the detected signal Sd corresponding to the time segment in which the first oscillation signal S1o and the second oscillation signal S2o have been received from the main control unit 172 and thereby extracts the first time segment data D1 and the second time segment data D2, respectively. Accordingly, in the same manner as in the case of the first embodiment, for the detected signal Sd, it is possible to refine data to only the data on the time segments during which the first pulsed laser beam LB1 and the second pulsed laser beam LB2 have been emitted, respectively.

Next, the quantity calculation unit 174 calculates the quantities of a lactic acid or lactic acid salt and a pyruvic acid in the measuring target substance MM to the blood BL based on the extracted first time segment data D1 and second time segment data D2. Specifically, the extracted first time segment data D1 includes, as an example thereof, two levels of output values, namely, the reference data D1s when the measuring target substance MM is not detected (the non-detection time segment Tn) and the detection data D1d when the measuring target substance MM is detected (the detection time segment Td).

Herein, in the same manner as in the case of the first embodiment, it can be determined that the larger the absolute value of the difference ΔD between the reference data D1s and the detection data D1d is, the greater the detected amount of the measuring target substance MM is. Accordingly, after the measurement for all the measurement ranges of interest, the quantity calculation unit 174 accumulates the number of detection data D1d in the entire first time segment data D1 and outputs the accumulated result to the main control unit 172 as the "quantity" of the measuring target substance MM.

On the other hand, the extracted second time segment data D2 includes, as an example thereof, two levels of output values, namely, reference data D2s when the measuring target substance MM is not detected (a non-detection time segment Tn) and detection data D2d when the measuring target substance MM is detected (a detection time segment T2d). Therefore, in the same manner as in the case of the first time segment data D1, it can be determined that the larger the absolute value of the difference ΔD2 between the reference data D2s and the detection data D2d is, the greater the detected amount of the measuring target substance MM is.

As described above, the biological information detection device 100 according to the third embodiment detects the reflected beams RB of the pulsed laser beam LB for the measuring target substance MM, extracts them as the first time segment data D1 and the second time segment data D2 corresponding to the first pulsed laser beam LB1 and the second pulsed laser beam LB2, and therefore can separately calculate the quantities of the lactic acid or lactic acid salt and the pyruvic acid in the measuring target substance MM. In such a way, it is performed detection and calculation by using the reflected beam RB of the pulsed laser beam LB and thus possible to employ a configuration of a measuring unit with a more compact size as a modified example for the third embodiment.

That is, as illustrated in Fig. 12, as a measuring unit 380, a configuration including, in an integrated manner, a cylindrical housing part 382 that contains a part of an organism including a blood vessel in which the blood BL flows, such as the finger 240, for example, the irradiation mechanism 130 that emits the pulsed laser beam LB toward the internal space S of the housing part 382, and the light receiving sensor 360 that detects a reflected beam of the pulsed laser beam LB can be indicated as an example. Accordingly, for the amount of light caused by a surrounding environment of the measuring unit 380, since the detected amount at the light receiving sensor 360 can be minimized, the measurement accuracy can be further increased.

With the configuration as described above, in addition that the advantageous effects described in the first embodiment can be obtained, the biological information detection device and the biological information detection method according to the third embodiment can minimize the detection of the amount of light that may be caused by the surrounding environment and therefore further increase the measuring accuracy by using a scheme to measure a reflected beam from a measuring target substance contained in blood.

Note that the present invention is not limited to the embodiments described above and can be changed as appropriate within the scope not departing from the spirit of the invention. In the present invention, modification of any component in the embodiment or omission of any component in the embodiment is possible within the scope of the invention. For example, the specific examples illustrated in the first embodiment to the third embodiment may be applied in combination of respective features.

### [List of Reference Symbols]

- 100: biological information detection device
- 110: first laser oscillator
- 112: oscillation control unit
- 113: drive power source
- 114: support part
- 115a, 115b: laser source
- 116: condenser lens
- 117: wavelength adjustment unit
- 118: transmission path
- 120: second laser oscillator
- 128: transmission path
- 130: irradiation mechanism
- 131a, 131b: connector
- 132: beam splitter
- 133, 134, 135: mirror
- 136: condenser lens
- 137: window member
- 140: transport mechanism
- 150: sample holding mechanism
- 152: container
- 160: light receiving sensor
- 170: control unit
- 172: main control unit
- 174: quantity calculation unit
- 176: display unit
- 178: input interface
- 240: finger
- 242: blood vessel
- 360: light receiving sensor
- 380: measuring unit
- 382: housing part

## Claims

1. A biological information detection device comprising:
a first laser oscillator that oscillates a first pulsed laser beam having a suitable wavelength adapted for detection of a lactic acid or lactic acid salt;
a second laser oscillator that oscillates a second pulsed laser beam having a suitable wavelength adapted for detection of a pyruvic acid;
an irradiation mechanism that emits the first pulsed laser beam and the second pulsed laser beam to blood inside which the lactic acid or lactic acid salt and the pyruvic acid are present;
a light receiving sensor that receives a detected laser beam output from the blood; and
a control unit that controls operations of respective components,
wherein the control unit outputs oscillation instructions for oscillating the first pulsed laser beam and the second pulsed laser beam at a constant cycle, respectively, to the first laser oscillator and the second laser oscillator, cuts out detected signals provided from the light receiving sensor as time segment data for time periods corresponding to the constant cycle, and calculates respective quantities of the lactic acid or lactic acid salt and the pyruvic acid in the blood based on the time segment data.

2. The biological information detection device according to claim 1, wherein the suitable wavelength of the first pulsed laser beam is 1480 nm, and the suitable wavelength of the second pulsed laser beam is 1462 nm.

3. The biological information detection device according to claim 1 or 2, wherein the first pulsed laser beam and the second pulsed laser beam are both emitted toward the blood that is flowing.

4. The biological information detection device according to any one of claims 1 to 3, wherein the light receiving sensor is configured to receive reflected beams of the first pulsed laser beam and the second pulsed laser beam from the blood, respectively.

5. A biological information detection method comprising: emitting a first pulsed laser beam, which has a suitable wavelength adapted for detection of a lactic acid or lactic acid salt, and a second pulsed laser beam, which has a suitable wavelength adapted for detection of a pyruvic acid, to blood inside which the lactic acid or lactic acid salt and the pyruvic acid are present; receiving detected laser beams output from the blood at a light receiving sensor; and acquiring quantities of the lactic acid or lactic acid salt and the pyruvic acid in the blood, respectively,
wherein the first pulsed laser beam and the second pulsed laser beam are oscillated at a constant cycle, respectively, and the biological information detection method comprises: cutting out, for each of the suitable wavelength of the first pulsed laser beam and the suitable wavelength of the second pulsed laser beam, detected signals provided from the light receiving sensor as time segment data for time periods corresponding to the constant cycle; and calculating respective quantities of the lactic acid or lactic acid salt and the pyruvic acid in the blood based on the time segment data.

6. The biological information detection method according to claim 5, wherein the suitable wavelength of the first pulsed laser beam is 1480 nm, and the suitable wavelength of the second pulsed laser beam is 1462 nm.

7. The biological information detection method according to claim 5 or 6, wherein the first pulsed laser beam and the second pulsed laser beam are both emitted toward the blood that is flowing.

8. The biological information detection method according to any one of claims 5 to 7, wherein as the detected laser beams, reflected beams of the first pulsed laser beam and the second pulsed laser beam are received from the blood, respectively.
